# EUROPÄISCHE PATENTSCHRIFT

(11) **EP 4 362 669 B1**
(45) Veröffentlichungstag und Bekanntmachung des Hinweises auf die Patenterteilung: **30.07.2025**
(21) Anmeldenummer: 22731480.4
(22) Anmeldetag: 09.06.2022
(51) Int. Cl.: A01K 67/36

(54) **VORRICHTUNG UND VERFAHREN ZUR AUFZUCHT VON INSEKTEN IN EINEM HOCHREGALLAGER**
DEVICE AND METHOD FOR REARING INSECTS IN A HIGH-BAY WAREHOUSE
DISPOSITIF ET PROCÉDÉ D'ÉLEVAGE D'INSECTES DANS UN MAGASIN A RAYONNAGES HAUTS

(30) Priorität: 02.07.2021 DE 102021117134
(43) Veröffentlichungstag der Anmeldung: 08.05.2024
(73) Patentinhaber: Alpha-Protein GmbH, 76646 Bruchsal (DE)
(72) Erfinder: SHALATI, Rohi, 76185 Karlsruhe (DE)
(74) Vertreter: Christ, Niko
(86) Internationale Anmeldenummer: PCT/DE2022/100435
(87) Internationale Veröffentlichungsnummer: WO 2023/274447

(56) Entgegenhaltungen:
- DE-A1- 102018 009 529
- DE-A1- 102020 005 146
- US-A1- 2012 123 141
- US-A1- 2018 070 566

## Beschreibung

Die Erfindung betrifft eine Vorrichtung zur Aufzucht von Insekten in einem Hochregallager, umfassend einen Lagerbereich mit wenigstens einem Hochregal und einer lagerlogistischen Einrichtung, wobei in dem wenigstens einen Hochregal Kisten stapelbar lagerbar sind und die Kisten mittels der lagerlogistischen Einrichtung innerhalb des Lagerbereichs transportierbar sind, wobei sich in den Kisten Insekten befinden. Auch betrifft die Erfindung ein Verfahren zur Aufzucht von Insekten in einem Hochregallager mit einer erfindungsgemäßen Vorrichtung.

Eine Vorrichtung zur Aufzucht von Insekten ist beispielsweise aus dem deutschen Patent DE 10 2019 121 102 B3 vorbekannt. Die Vorrichtung ermöglicht eine automatisierte Aufzucht von Insekten, bei der mittels eines Portals ein Stapel von Kisten nach einem Paternosterprinzip umgewälzt und die innerhalb der Kisten befindlichen Insekten durch ein Versorgungsmodul versorgt werden.

Weiterhin ist aus der internationalen Patentanmeldung WO 2016/153340 A2 eine Vorrichtung und ein Verfahren zur Aufzucht von Insekten offenbart. Die Vorrichtung umfasst dabei
- einen Laichbereich, in dem eine Vielzahl von Laichbehältern vorhanden ist, die angepasst sind, um erwachsene Insekten einschließlich Mutterinsekten und Insektenfutter aufzunehmen oder aufgenommen zu haben, wobei die Vielzahl von Laichbehältern in einem oder mehreren Stapeln von Laichbehältern gestapelt ist;
- eine Vielzahl von Laichstrukturen, wobei mindestens eine Laichstruktur angepasst ist, um in jedem Laichbehälter in dem Laichbereich bereitgestellt zu werden oder bereitgestellt ist, wobei die Mutterinsekten in diesen Laichstrukturen ihre Eier ablaichen werden, so dass die Laichstruktur oder ein Teil der Laichstruktur die Eier hält, wobei die Laichstruktur oder der Teil davon aus dem Laichbehälter entfernt werden kann, wobei die erwachsenen Insekten in dem Laichbehälter verbleiben;
- einen Schlupfbereich, in dem die Eier schlüpfen werden, wobei der Schlupfbereich so angepasst ist, dass er die Laichstruktur oder den Teil davon, der die Eier hält, aufnimmt und aus den Laichbehältern entfernt werden kann, was das Ernten der Baby-Larven ermöglicht;
- ein Nahrungszufuhrsystem zum Zuführen von Nahrung zu den Laichbehältern;
- einen Bereich zum Handhaben von Laichstrukturen, der ein System zum Handhaben von Laichstrukturen umfasst, das angepasst ist, um die Laichstruktur oder den Teil davon, der die Eier hält, aus dem Laichbehälter zu entfernen und die Laichstruktur oder den Teil davon zu dem Schlupfbereich zu transportieren und eine leere Laichstruktur oder einen Teil davon in jedem Laichbehälter bereitzustellen, wobei der Bereich zum Handhaben von Laichstrukturen vorzugsweise auch angepasst ist, um die Laichstrukturen oder Teile davon zu reinigen;
- sowie ein Behälterhandhabungssystem zum Transportieren der Laichbehälter zwischen dem Laichbereich und dem Laichstruktur-Handhabungsbereich.

Ferner ist aus der US 2018/0070566 A1 eine Farm zur Aufzucht von Insekten vorbekannt, wobei die Erfindung eine Anlage zur Aufzucht von Insekten betrifft, die einen ersten Bereich, in dem die aufzuziehenden Insekten in Behältern gelagert werden, während sie wachsen, und einen zweiten Bereich umfasst, der mindestens eine Station umfasst, die so konfiguriert ist, dass sie eine auf die Aufzucht bezogene Aufgabe an den Insekten in einem Behälter oder an dem Behälter durchführt. Die Behälter sind in der ersten Zone in Sätzen von palettierten Behältern gruppiert, die als Grundeinheiten bezeichnet werden. Die erste Zone umfasst dabei Palettenbahnen, in denen die Grundeinheiten angeordnet sind. Die erste Zone ist außerdem mit einer automatischen Vorrichtung ausgestattet, die so konfiguriert ist, dass sie die Grundeinheiten zwischen der ersten Zone und einer Schnittstelle mit der zweiten Zone bewegt.

Weiterhin ist aus der internationalen Patentanmeldung WO 2014/171829 A1 ein Verfahren und ein System zum Züchten von Insekten unter Verwendung einer Vielzahl von Einzelkisten vorbekannt, bei dem zumindest ein Teil jeder Kiste mit einem Substrat gefüllt ist, das Futtermittel und unreife Phasen von Insekten enthält. Außerdem ist ein Klimaraum vorgesehen, in dem die Kästen untergebracht sind und der ein Belüftungssystem aufweist. Ein Fördersystem ist in dem System enthalten, um die Kästen aus dem Klimaraum zu holen und dorthin zurückzubringen. Entlang des Fördersystems sind ein Beobachtungssystem zur Gewinnung von Beobachtungen, einschließlich Daten und Messungen, und stromabwärts davon eine Zufuhrstation für das Ausgangsmaterial angeordnet.

Auch ist aus der internationalen Patentanmeldung WO 2017/223096 A1 eine autonome Futterlieferungsplattform vorbekannt, die so konfiguriert ist, dass sie durch eine Anlage navigiert und Insektenfutter an mehrere Insektenhabitate liefert, die sich innerhalb der Anlage befinden. In einigen Fällen kann die Futterlieferplattform so konfiguriert sein, dass sie das Futter an mehrere Insektenhabitate im Wesentlichen zur gleichen Zeit liefert.

Ferner ist aus der DE 10 2020 005 146 A1 ein Verfahren zur industriellen Zucht der schwarzen Soldatenfliege und zur Weiterverarbeitung ihrer Larven zu Insektenmehl sowie eine Einrichtung hierfür offenbart. Auch in der DE 10 2018 009 529 A1 wird die Aufzucht von Insektenlarven offenbart, die anschließend zu einem Ernährungsmittel oder einer Ernährungsmittelkomponente verarbeitet werden.

Letztlich offenbart die US 2012/0123141 A1 ein Verfahren zur Extraktion von ungesättigten Ölen aus Insektenlarven.

Ausgehend von diesem Stand der Technik stellt sich die Erfindung die Aufgabe, eine automatisierte, ressourcenschonende und effiziente Aufzucht von Insekten in einem Hochregallager bereitzustellen. Diese Aufgabe wird mit einer Vorrichtung gemäß des unabhängigen Anspruchs 1 gelöst. Weiter wird diese Aufgabe mit einem Verfahren gemäß dem nebengeordneten Anspruch 17 gelöst. Vorteilhafte Ausgestaltungen der Erfindung können den abhängigen Ansprüchen entnommen werden.

Bei der Erfindung handelt es sich in einem Aspekt um eine Vorrichtung zur Aufzucht von Insekten in einem Hochregallager, umfassend einen Lagerbereich mit wenigstens einem Hochregal und einer lagerlogistischen Einrichtung, wobei in dem wenigstens einen Hochregal Kisten stapelbar lagerbar sind und die Kisten mittels der lagerlogistischen Einrichtung innerhalb des Lagerbereichs transportierbar sind, wobei sich in den Kisten Insekten befinden. Damit die zu transportierenden Kisten unter anderem nur möglichst kurze Wegstrecken zurücklegen müssen, sieht die erfindungsgemäße Vorrichtung vor, dass an dem wenigstens einen Hochregal ein Versorgungsportal angeordnet ist, zu welchem die lagerlogistische Einrichtung die in dem wenigstens einem Hochregal stapelbar gelagerten Kisten transportieren kann, wobei der Zustand des Kisteninhalts innerhalb der vom lagerlogistischen Einrichtung zum Versorgungsportal transportierten Kisten mittels des Versorgungsportals bestimmt werden kann und die Kisten mittels des Versorgungsportals versorgt und/oder bei erntereife der Insekten die Kisten ausgeschleust werden können. Die Bestimmung des Zustandes des Kisteninhalts kann dabei beispielsweise durch Kennzeichnung einer jeden Kiste erfolgen, wobei die Kennzeichnung vom Versorgungsportal ausgelesen und anhand der hiermit verbundenen Daten, beispielsweise der Dauer des Lagerzeitraums im Hochregallager, das Versorgungsportal entscheiden kann, ob und in welcher Form die Kiste gefüllt mit Insekten versorgt und/oder ausgeschleust wird. Durch die von der lagerlogistischen Einrichtung kurzen, zurückzulegenden Wegstrecken der Kisten wird eine erhöhte Versorgung der im Wachstum befindlichen Insekten ermöglicht. Somit kann letztlich eine optimierte Aufzucht der innerhalb der Kisten befindlichen Insekten gewährleistet werden.

Dabei kann die lagerlogistische Einrichtung ein Regalbediengerät, ein Shuttle oder ein Durchlaufregal zur Transportation der Kisten aus dem wenigstens einem Hochregal zum Versorgungsportal sein.

Auch können sich innerhalb der Kisten nicht nur die aufzuziehenden Insekten, sondern vielmehr auch Insektenhäute, Insekteneier, Kot, Futtermittel, Substrat und sonstiger Kisteninhalt befinden, die letztlich auch einen Einfluss auf die erfolgreiche und effiziente Aufzucht der Insekten haben. Weiterhin können die Kisten sowohl mit unterschiedlichen Insektenarten als auch mit unterschiedlichen Stadien der Insekten gefüllt sein. Abhängig von der Insektenart, insbesondere bei Heuschrecken, findet dabei keine gestapelte Lagerung im Hochregal statt, da sich die zu verwendende Art von Kiste nicht zur Stapelung eignet.

In einer ersten vorteilhaften Ausgestaltung weist das Versorgungsportal wenigstens einen Pufferbereich auf, in welchem die von der lagerlogistischen Einrichtung zum Versorgungsportal transportierten Kisten zwischenlagerbar sind. Im Ergebnis kann hierdurch die Effizienz weiter gesteigert werden.

Um feststellen zu können, ob in den Kisten insbesondere das optimale Klima bzw. die optimale Bedingung für die Aufzucht vorherrscht, sieht die Vorrichtung in einer weiteren vorteilhaften Ausgestaltung vor, dass das Versorgungsportal wenigstens eine Detektionseinrichtung aufweist, mit welcher die für die Insektenzucht benötigten Parameter, insbesondere Feuchtigkeit und Temperatur, innerhalb einer Kiste feststellbar sind. Damit kann im Ergebnis eine effizientere Aufzucht der Insekten gewährleistet werden, da die qualitativen, physiologischen und pathologischen Zustände des Kisteninhalts bestimmt werden können. In einer abermals vorteilhaften Ausgestaltung verfügt die Detektionseinrichtung über wenigstens einen Sensor und/oder eine Vorrichtung zur Aufzeichnung und/oder Datenübermittlung, insbesondere eine Kamera, um die entsprechenden Parameter bzw. den Zustand des Kisteninhalts, insbesondere der Insekten, innerhalb einer Kiste feststellen zu können. Insbesondere durch den Einsatz einer Kamera besteht die Möglichkeit ein Gesamtbild des Zustandes der Insekten und des restlichen Kisteninhalts innerhalb einer Kiste feststellen zu können.

Dabei können in einer vorteilhaften Ausgestaltung der Vorrichtung die mittels der Detektionseinrichtung festgestellten Parameter, die letztlich den Zustand des Kisteninhalts, insbesondere der Insekten, in einer der Kisten widerspiegeln, mit einer Referenzdatenbank verglichen werden, sodass der jeweilige Zustand des Kisteninhalts und/oder der Reifegrad der Insekten feststellbar ist. Die so durch die Detektionseinrichtung erhaltenen Daten können weiterhin zum Aufbau einer Referenzdatenbank sowie durch Vergleich der in der Referenzdatenbank hinterlegten Daten mit jeweils aktuell bestimmten Parameterdaten zum maschinellen Lernen der Detektionseinrichtung dienen. Letztlich können die so erhaltenen Daten bevorzugt zur automatisierten Optimierung der Produktionsparameter, insbesondere der Verbesserung der Rezeptur und Dosierung des Futtermittels, herangezogen werden. Im Ergebnis kann somit eine weitere Optimierung der Aufzucht von Insekten erreicht werden.

Weiterhin weist das Versorgungsportal in einer vorteilhaften Ausgestaltung der Vorrichtung wenigstens ein Versorgungsmodul auf, mit welchem die Insekten innerhalb der vom Regalbediengerät in das Versorgungsportal transportierten Kisten mit Versorgungsmaterialien versorgbar sind. Insbesondere fügt das Versorgungsmodul den Kisten Futtermittel und Substrat hinzu, um das Wachstum der innerhalb der Kisten befindlichen Insekten voranzutreiben.

Um unerwünschte Nebenprodukte der Insekten, insbesondere Insektenhäute, Kot sowie ungefressene Futterreste, wobei Letzteres verderben und somit eine potentielle Gefahr für die Erkrankung der Insekten darstellen kann, entfernen zu können, sieht die Vorrichtung in einer weiteren vorteilhaften Ausgestaltung vor, dass das Versorgungsportal über wenigstens ein Reinigungsmodul verfügt. Mit diesem Reinigungsmodul kann das Versorgungsportal sonstigen Kisteninhalt, insbesondere Häute der Insekten, Kot und Futtermittelreste, aus der Kiste entfernen und somit ebenfalls zu einer gesteigerten Wachstumsrate der Insekten beitragen. In einer abermals vorteilhaften Ausgestaltung weist das Reinigungsmodul wenigstens eine Saugvorrichtung auf, mit welcher durch Absaugen oder Windsichten der sonstige Kisteninhalt aus einer Kiste entfernbar ist. Auch kann die Entfernung des sonstigen Kisteninhalts, insbesondere der Insektenhäute und des Kotes der Insekten, durch wenigstens eine am Reinigungsmodul vorhandene elektrische Einrichtung erfolgen, mit welcher durch Elektrostatik der entsprechende sonstige Kisteninhalt aus einer Kiste entfernbar ist.

Weiterhin sieht die Vorrichtung in einer vorteilhaften Ausgestaltung vor, dass zur Optimierung der automatisierten Aufzucht von Insekten in einem Hochregallager die erfindungsgemäße Vorrichtung wenigstens einen Schlüpfbereich aufweist. In diesem Schlüpfbereich finden Schlüpfprozesse statt, insbesondere können von reproduzierenden Insekten gelegte Insekteneier und/oder -puppen in diesem Bereich schlüpfen. Aber auch anderweitig beschaffte Insekteneier oder -puppen können in dem Schlüpfbereich gebrütet werden. Die Insekteneier und/oder -puppen können dabei in Kisten, flachen Kisten und/oder flachen Tablets angeordnet sein. Sobald insbesondere die Larven und/oder Insekten aus den Insekteneiern und/oder -puppen geschlüpft sind, können diese in einer weiteren vorteilhaften Ausgestaltung der Vorrichtung in einem Puppenbearbeitungsbereich weiter bearbeitet werden. Somit können die zur Aufzucht vorgesehenen Insekten selbstständig reproduziert und anschließend beispielsweise durch ein geeignetes Transportsystem und der im Lagerbereich vorhandenen lagerlogistischen Einrichtung zur weiteren Aufzucht in das wenigstens eine Hochregal, an welches ein Versorgungsportal angeordnet ist, überführt werden.

Um die optimalen Bedingungen zum Brüten der Insekteneiern und/oder - puppen und damit letztlich eine gesteigerte Anzahl von geschlüpften Larven und/oder Insekten zu erzielen, sieht die Vorrichtung in einer weiteren vorteilhaften Ausgestaltung vor, dass der Schlüpfbereich einen Inkubator zum Brüten der Insekteneier und/oder -puppen aufweist. So kann sichergestellt werden, dass die optimalen Bedingungen für das Schlüpfen der Larven und/oder Insekten aus den Insekteneiern und/oder -puppen vorherrschen, um im Ergebnis die Effizienz der Insektenaufzucht weiter zu steigern.

Ferner sieht die Erfindung für die Vorrichtung in einer vorteilhaften Ausgestaltung vor, dass diese wenigstens eine Separationseinrichtung aufweist, mit welchen die Insekteneier und/oder -puppen oder die im Schlüpfbereich, insbesondere im Inkubator geschlüpften Larven und/oder Insekten sowie im Puppenbearbeitungsbereich bearbeiteten Insektenpuppen, Larven oder Insekten vom Rest des Kisteninhalts abtrennbar sind. Somit ist es möglich, eventuell noch nicht geschlüpfte Eier und/oder Puppen von den bereits aus den Insekteneiern und/oder Insektenpuppen geschlüpften Larven und/oder Insekten zu trennen und die so geschlüpften Larven und/oder Insekten aus dem Schlüpfbereich bzw. Puppenbearbeitungsbereich zur weiteren Aufzucht in das wenigstens eine Hochregal im Lagerbereich und die gegebenenfalls noch nicht geschlüpften Eier oder Puppen zurück in den Schlüpf- oder Puppenbearbeitungsbereich zur weiteren Behandlung zu überführen. Auch können bereits im Schlüpf- oder Puppenbearbeitungsbereich angefallene Rückstände der aus den Insekteneiern und/oder -puppen geschlüpften Larven und/oder Insekten sowie die zurückbleibenden Eierschalen oder Puppen durch die Separationseinrichtung entfernt werden.

Um die Behältnisse zur Befüllung mit Insekteneiern vorbereiten bzw. um die im Inkubator aus den Insekteneiern und/oder Insektenpuppen geschlüpften Larven und/oder Insekten versorgen zu können und um dabei beispielsweise ein Transportmittel oder ein Fördersystem möglichst kurze Wegstrecken zurücklegen zu lassen, sieht die Vorrichtung in einer weiteren vorteilhaften Ausgestaltung vor, dass diese wenigstens eine Versorgungseinrichtung aufweist, mit dem das mit Insekteneier und/oder Insektenpuppen befüllte Behältnis mit entsprechenden Versorgungsmaterialien befüllbar ist. Dies hat den Vorteil, dass alle für die komplette Reproduktion erforderlichen Schritte, ausgehend von dem Brüten der Insekteneier und/oder Insektenpuppen bis zur (Erst-)Versorgung der geschlüpften Larven und/oder Insekten sowie dem Einbringen dieser in eine Kiste für die weitere Aufzucht, innerhalb eines Bereichs durchführbar sind.

Sind die innerhalb der Kisten befindlichen Insekten erntereif bzw. haben den für die weitere Verarbeitung erforderlichen Reifegrad erreicht, müssen diese innerhalb des wenigstens einen Hochregal stapelbar gelagerten Kisten entstapelt, der Kisteninhalt aus den in Kisten entfernt und die Kisten gereinigt werden. Um diese Aufgabe zu erfüllen, sieht die Vorrichtung in einer vorteilhaften Ausgestaltung vor, dass diese wenigstens einen Entleerungsbereich aufweist, in dem die zuvor genannten Schritte durchführbar sind.

Dabei weist der Entleerungsbereich in einer weiteren vorteilhaften Ausgestaltung wenigstens eine Entleerungseinrichtung auf, mit welchem die Entstapelung der Kisten und die Entfernung des Kisteninhalts aus den Kisten durchführbar ist. Damit kann eine schnelle und schonende Entleerung der mit Insekten befüllten, stapelbaren Kisten erreicht werden.

Um die im Entleerungsbereich entstapelten und entleerten Kisten wieder verwenden zu können, sieht die Vorrichtung in einer abermals vorteilhaften Ausgestaltung vor, dass der Entleerungsbereich wenigstens eine Reinigungseinrichtung aufweist, mit welcher die entleerten und entstapelten Kisten reinigbar sind.

Damit der aus den Kisten entleerte Kisteninhalt im Entleerungsbereich aufgetrennt werden kann, sieht die Vorrichtung in einer vorteilhaften Ausgestaltung vor, dass wenigstens ein Separationsbereich vorhanden ist, in welchem der aus den Kisten entleerte Kisteninhalt in Produkte, Zwischenprodukte und sonstigem Kisteninhalt auftrennbar ist. Produkte können hierbei insbesondere erntereife Insekten einer gewünschten Größe, Zwischenprodukte noch nicht vollends ausgereifte Insekten und sonstiger Kisteninhalt beispielsweise Futtermittelreste, Kot und Insektenhäute sein. In einer abermals vorteilhaften Ausgestaltung kann der aus den Kisten entleerte Kisteninhalt in Form eines Schüttguts zu dem Separationsbereich transportiert werden.

In einer abermals vorteilhaften Ausgestaltung sieht die Vorrichtung vor, dass die mittels der Reinigungseinrichtung im Entleerungsbereich gereinigten und entleerten Kisten in wenigstens einem Pufferbereich gelagert und für die weitere Verwendung vorgehalten werden können.

Weiterhin sieht die Vorrichtung in einer vorteilhaften Ausgestaltung vor, dass diese wenigstens einen Befüllungsbereich aufweist, in welchem die Kisten mit Futtermittel, Insekten, Insekteneiern, Substraten oder einer Kombination hiervon befüllbar sind. Der Befüllungsbereich dient wesentlich zu einer Erstbestückung bzw. Erstbefüllung oder Wiederbefüllung der Kisten mit den für die Aufzucht wesentlichen Materialien, insbesondere Substrat, Insekteneier, Larven und Futtermittel. Auch können mittels des Befüllungsbereichs Kisten mit zur Aufzucht vorgesehenen Insekten, Insektenlarven oder frisch geschlüpften Insekten erstmalig befüllt und mit entsprechenden Materialien für die weitere Aufzucht versorgt werden. Bevorzugt verfügt der Befüllungsbereich über wenigstens ein Versorgungsportal zur Erstbestückung bzw. Wiederbefüllung der für die Aufzucht vorgesehenen Kisten. Besonders bevorzugt weist der Befüllungsbereich für wenigstens ein vorhandenes Stadium der Insekten, insbesondere für Puppen, frisch geschlüpfte Larven oder Käfer, jeweils ein Versorgungsportal auf.

Weiterhin sieht die Vorrichtung in einer vorteilhaften Ausgestaltung vor, dass der Befüllungsbereich über wenigstens eine Befüllungsstraße verfügt, in welcher die Befüllung der Kisten stattfindet. Dabei kann die Befüllungsstraße unterschiedliche Stationen bzw. Module aufweisen, die die zu befüllende Kiste mit den nötigen Materialen ausstattet.

Damit die im Lagerbereich in dem wenigstens einen Hochregal stapelbar gelagerten und durch die lagerlogistische Einrichtung transportierbaren Kisten aus und in den Lagerbereich transportierbar sind, sieht die Vorrichtungen in einer vorteilhaften Ausgestaltung vor, dass diese wenigstens ein Fördersystem aufweist, mit welchen die Kisten aus und in den Lagerbereich beförderbar sind. In einer abermals vorteilhaften Ausgestaltung sieht die Vorrichtung vor, dass es sich bei dem Fördersystem ganz oder teilweise um ein fahrerloses Transportsystem handelt. Bei einem fahrerlosen Transportfahrzeug handelt es sich um ein flurgebundenes Fördermittel mit eigenem Fahrantrieb, dass automatisch gesteuert und berührungslos geführt wird. Diese fahrerlosen Transportfahrzeuge dienen üblicherweise dem Materialtransport, und zwar zum Ziehen oder Tragen von Fördergut mit aktiven oder passiven Lastaufnahmemitteln, nicht aber dem Personentransport. Solche Transportsysteme bestehen im Wesentlichen aus einem oder mehreren fahrerlosen Transportfahrzeugen, einer Leitsteuerung, einer Einrichtung zur Standortbestimmung und Lageerfassung, einer Einrichtung zur Datenübertragung sowie einer Infrastruktur und einer peripheren Einrichtung.

Weiterhin sieht die Vorrichtung in einer abermals vorteilhaften Ausgestaltung vor, dass diese wenigstens einen Anlieferungsbereich aufweist, an welche Materialien, insbesondere Futtermittel, Rohstoffe für Futtermittel, Substrat oder etwaige weitere zur Aufzucht von Insekten notwendige Materialien, anlieferbar sind.

Bevorzugt kann an den Anlieferungsbereich wenigstens ein Futterrohstoffsilo angeordnet sein, in dem unter anderem Rohstoffe zur Futtermittelherstellung gelagert und für die weitere Verwendung vorgehalten werden können.

Auch kann die Vorrichtung in einer weiteren vorteilhaften Ausgestaltung wenigstens ein Produktsilo aufweisen, in dem ein aus der Aufzucht gewonnenes Produkt, beispielsweise lebende Insekten zum Einsatz als Futtermittel für Tiere, gelagert werden können.

Um die für das Wachstum optimalen Bedingungen für die Insekten innerhalb der im wenigstens einen Hochregal stapelbar gelagerten Kisten sicherzustellen, sieht die Vorrichtung in einer weiteren vorteilhaften Ausgestaltung vor, dass der Lagerbereich wenigstens eine Klimatisierungseinrichtung aufweist, mit welcher die Luftqualität innerhalb des Lagerbereichs reguliert wird. Unter der Luftqualität ist hierbei unter anderem die Temperatur, die Luftfeuchtigkeit sowie die Partikelkonzentration innerhalb der Luft zu verstehen. Somit können mittels der Klimatisierungseinrichtung die optimalen Bedingungen für ein effizientes Wachstum der Insekten eingestellt werden.

Auch ist es von Vorteil, wenn innerhalb des Lagerbereichs ein Zugang an dem wenigstens einen, am Hochregal angeordneten Versorgungsportal vorhanden ist, sodass die Vorrichtung in einer vorteilhaften Ausgestaltung vorsieht, dass innerhalb des Lagerbereichs an dem wenigstens einen Hochregal angeordneten Versorgungsportal wenigstens eine Plattform zur Wartung, zum manuell Eingreifen und zum manuellen Bestücken des Versorgungsportal vorhanden ist.

Weiterhin sieht die Vorrichtungen in einer abermals vorteilhaften Ausgestaltung vor, dass der Lagerbereich in Silobauweise ausgeführt ist, sodass das wenigstens eine Hochregal als Tragelement für wenigstens eine Außenfassade dient. Hierdurch kann im Ergebnis eine Materialeinsparung erreicht werden, womit letztlich wiederum eine ressourcenschonende und effiziente Aufzucht von Insekten gewährleistet werden kann. In einer weiteren vorteilhaften Ausgestaltung sieht die Vorrichtung vor, dass das wenigstens eine als Tragelement und Außenfassade dienende Hochregal eine Isolierung mit einer Dampfsperre aufweist, sodass der Lagerbereich nach außen hin abgeschirmt ist. Somit kann insbesondere sichergestellt werden, dass eine Änderung der klimatischen Bedingungen im Außenbereich kaum bis keine Auswirkungen auf die innerhalb des Lagerbereichs vorherrschenden Bedingungen hat.

In einem zweiten Aspekt handelt es sich bei der Erfindung um ein Verfahren zur Aufzucht von Insekten mit einer erfindungsgemäßen Vorrichtung, bei der ein Stapel von Kisten aus wenigstens einem Hochregal entnommen und zu einem Versorgungsportal transportiert wird, wobei für jede der in das Versorgungsportal transportierten Kiste der Zustand des Kisteninhalts der jeweiligen Kiste mittels des Versorgungsportals bestimmt und abhängig von dem bestimmten Zustand des Kisteninhalts die Kiste ausgeschleust oder versorgt wird, wobei nach Versorgung die jeweiligen Kisten in das wenigstens eine Hochregal zurück transportiert werden. Die Versorgung kann dabei vom Zustand des Kisteninhalts, insbesondere abhängig vom Alter, dem Besatz, dem Lebensstadium der Insekten, der Charge und weiteren Faktoren abhängig gemacht werden. Hierdurch kann eine automatisierte und effiziente Aufzucht von Insekten in einem Hochregallager erreicht werden.

Um die Effizienz des Verfahrens zur Aufzucht von Insekten in einem Hochregallager weiter zu erhöhen, sieht das Verfahren in einer vorteilhaften Ausgestaltung vor, dass die Lagerung der stapelbaren Kisten im wenigstens einen Hochregal nicht nach einem Festplatzsystem gelagert werden, sondern nach dem Chaosprinzip oder einer anderen optimierten Lagerform, wobei es sich bei dem Chaosprinzip um eine sogenannte dynamische Lagerhaltung handelt, bei der ein einzulagerndes Teil keinen festen Lagerplatz im Lagerbereich aufweist, sondern auf beliebige, zum Zeitpunkt des Einlagerns, nicht belegte Plätze eingelagert wird. Im Ergebnis kann hiermit unter anderem eine Nivellierung des Maschinenutzungsgrades der Versorgungsportale, der lagerlogistischen Einrichtung und eine bessere Nutzung der Lagerfläche erreicht werden, sowie neue stapelbare Kisten leichter eingelagert und gewechselt werden.

Auch sieht das Verfahren in einer weiteren vorteilhaften Ausgestaltung vor, dass zu dem Versorgungsportal transportierte Kisten von diesem Versorgungsportal zu einem anderem Versorgungsportal transportiert werden. Dies kann beispielsweise durch die lagerlogistische Einrichtung selbst oder durch ein hierfür geeignetes Transportmittel geschehen. Dies ist dann von Vorteil, wenn die Kapazität des Versorgungsportals, zu welchem die Kisten transportiert wurden, ausgelastet ist oder das entsprechende Versorgungsportal defekt ist oder gewartet werden muss. Um eine Totzeit zu vermeiden, werden daher diese Kisten an ein weiteres vorhandenes Versorgungsportal transportiert, dessen Kapazität nicht voll ausgelastet ist, damit die Kisten dort entsprechend untersucht und gegebenenfalls ausgeschleust und/oder versorgt werden. Im Ergebnis wird somit eine schnellere Verarbeitung der Kisten gewährleistet und Totzeiten vermieden.

Weiterhin sieht das Verfahren vor, dass bei erntereifen Kisten das Substrat und/oder Futtermittel entfernt wird, sodass die Insekten ihren Darm entleeren können. Dies ist insbesondere deshalb von Vorteil, da hierdurch eine Reinigung des aus den Insekten gewonnen Rohprodukts vermieden werden kann, da das Rohprodukt hierdurch bereits von etwaigen noch in den Insekten vorhandenen Exkrementen nahezu vollständig befreit wird. Bevorzugt findet die Trennung 1 bis 8 Tage, besonders bevorzugt 1 bis 5 Tage vor der eigentlichen Ernte statt, also bevor die Kiste beispielsweise vom Versorgungsportal oder von der lagerlogistischen Einrichtung zur weiteren Verarbeitung aus dem Lagerbereich ausgeschleust wird. Dies deshalb, da hierdurch der Anteil an Nährstoffen im Produkt, also dem Insekt, gesteigert und die sonstigen Bestandteile, beispielsweise Wasser und Exkremente, verringert werden können.

Ferner sieht das Verfahren in einer abermals vorteilhaften Ausgestaltung vor, dass die erntereifen, vom Versorgungsportal oder der lagerlogistischen Einrichtung ausgeschleusten Kisten entstapelt und entleert werden. Auch können die entleerten Kisten in einer vorteilhaften Ausgestaltung des Verfahrens gereinigt werden. Weiterhin können die gereinigten Kisten gelagert und für die weitere Verwendung vorgehalten werden, insbesondere zur Aufnahme von Zwischenprodukten oder zur Neubefüllung.

Auch sieht das Verfahren vor, dass der aus den entleerten Kisten entstammende Kisteninhalt in Produkte, Zwischenprodukte und sonstigem Kisteninhalt separiert wird. Produkte können hierbei Insekten eines entsprechenden Gewichts oder Größe sein, wobei kleinere Insekten oder leichtere Insekten als Zwischenprodukte gelten können, die in einer weiteren vorteilhaften Ausgestaltung des Verfahrens zur weiteren Aufzucht wieder zurück in eine Kiste gefüllt werden können. Der sonstige Kisteninhalt, der beispielsweise aus toten Insekten, Insektenhäuten, Substrat- und/oder Futtermittelresten, Kot und sonstigen Bestandteilen bestehen kann, kann als sonstiger Kisteninhalt gesondert zur weiteren Vernichtung beziehungsweise Verarbeitung abgeleitet werden.

Um die nunmehr als Produkt geltenden Insekten weiterverarbeiten zu können, sieht das Verfahren in einer abermals vorteilhaften Ausgestaltung vor, dass die Produkte entweder als Lebendprodukt erhalten werden oder inaktiviert und geschreddert werden. Die Inaktivierung kann dabei insbesondere durch eine starke Temperaturänderung realisiert werden, wobei diese Erhitzung oder Kühlung, insbesondere durch Blanchieren oder Gefriertrocknen, erfolgen kann. Nach Inaktivierung kann das Produkt geschreddert und das so erhaltene geschredderte Produkt den weiteren Verarbeitungsschritten zugeführt werden.

In einer weiteren vorteilhaften Ausgestaltung sieht das Verfahren vor, dass das geschredderte Produkt entfettet und ein Öl aus dem Produkt gebildet wird. Das so erhaltene entfette Produkt kann anschließend in einer weiteren vorteilhaften Ausgestaltung des Verfahrens getrocknet und gemahlen werden, sodass letztlich ein Mehl gewonnen werden kann.

In einem weiteren Aspekt sieht die Erfindung die Verwendung eines aus dem Verfahren gewonnen Mehls für Futtermittel für die Insekten- und Tieraufzucht sowie für Lebensmittel, insbesondere in Form von Proteinpulver, vor.

Ferner sieht die Erfindung in einem weiteren Aspekt die Verwendung eines aus dem Entfetten gewonnen Öl für Lebensmittel- und Kosmetikprodukte sowie für Futtermittel vor. Beispielsweise kann das Öl als reines Öl zur Zubereitung von Dressings für Salate als auch in der Lebensmittelproduktion als Ersatz für Palmöl genutzt werden. Darüber hinaus ist auch der Einsatz in Kosmetikprodukte, insbesondere als Ersatz von Palmöl, denkbar. Letztlich kann das Öl selbst oder zur Herstellung von Futtermittel dienen.

Nachfolgend wird die Erfindung anhand eines Ausführungsbeispiels näher erläutert.

Es zeigt:

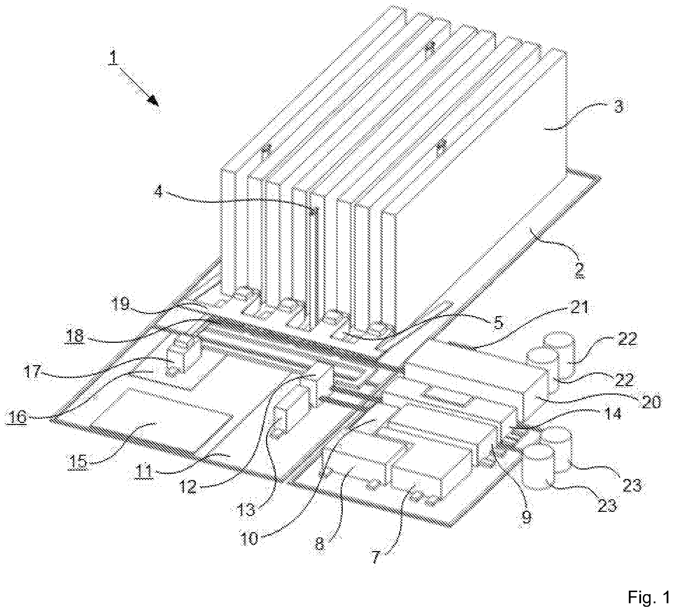

- Fig. 1:: eine schematische Darstellung einer Vorrichtung zur Aufzucht von Insekten in einer perspektivischen Ansicht.

Figur 1 zeigt eine schematische Darstellung einer Vorrichtung zur Aufzucht von Insekten in einer perspektivischen Ansicht. Im Lagerbereich 2 sind mehrere Hochregale 3 in Zweierreihen angeordnet, wobei zwischen den Zweierreihen sich ausbildenden Freiräumen ein Regalbediengerät 4 als lagerlogistische Einrichtung entlang fahrbar ist und das Regalbediengerät 4 Kisten vom und zu dem jeweiligen Hochregal 3 transportieren kann. Darüber hinaus können die Regalbediengeräte 4 aus den Hochregalen 3 heraus und zu den an jedem zweiten Hochregal 3 angeordneten Versorgungsportal 5 fahren und somit Kisten aus den Hochregalen 3 in die an jede Zweierreihe angeordneten Versorgungsportale 5 transportieren.

Die aus den Hochregalen 3 mittels der Regalbediengeräte 4 in die an den Zweierreihen angeordneten Versorgungsportalen 5 transportierten Kisten können innerhalb der Versorgungsportale 5 untersucht werden, wobei abhängig vom Zustand des Kisteninhalts das Versorgungsportal 5 die Kisten mit Versorgungsmaterialien, insbesondere Futter und Substrat, versorgen oder im Falle einer erntereifen Kisten diese aus dem Lagerbereich 2 ausschleusen kann. Ferner können erntereife Kisten auch direkt mittels des Regalbediengeräts 4 aus dem Lagerbereich 2 ausgeschleust werden. Dies kann beispielsweise aufgrund eines abgelaufenen Zeitintervalls, dass auf empirischen Daten beruht, geschehen. Somit ist es möglich, auch ohne Prüfung des Zustandes des Kisteninhalts durch ein Versorgungsportal 5 diese mittels des Regalbediengeräts 4 aus dem Lagerbereich 2 auszuschleusen.

Nach einer möglichen Versorgung der Kisten durch das Versorgungsportal 5 können diese wieder mittels des Regalbediengeräts 4 aus den Versorgungsportalen 5 entnommen und zurück in die Hochregale 3 zur weiteren Aufzucht gebracht werden, wobei die Lagerung bzw. Unterbringung der mittels des Versorgungsportals 5 versorgten Kisten beispielsweise nach dem Chaosprinzip oder einer anderen Form der optimierten Lagerhaltung erfolgen kann. Auch können die versorgten Kisten zunächst in einem am Versorgungsportal 5 vorhandenen Pufferbereich zwischengelagert werden, bis das Regalbediengerät 4 diese zurück in das Hochregal 3 bringen.

Für den Fall, dass die Kisten in Form von Stapeln oder Doppelstapel gelagert werden, ist es denkbar, dass das Versorgungsportal 5 über die notwendige Einrichtung verfügt, diese Kistenstapeln entsprechend zu entstapeln, um die entsprechende Untersuchung und Versorgung der Kisten durchzuführen.

Um gegebenenfalls manuell bei der automatisierten Versorgung der Kisten durch die Versorgungsportale 5 eingreifen zu können, befindet sich entlang der an den Hochregalen 3 angeordneten Versorgungsportale 5 eine Plattform 19, sodass beispielsweise Mitarbeiter manuell eine Probe aus den Kisten oder von den Versorgungsmaterialien entnehmen oder eine in Augenscheinnahme durchführen können.

Zur Erst- oder Neubestückung bzw. Wiederbefüllung von Kisten mit Insekten und den entsprechenden zur Zucht benötigten Materialien, weist die Vorrichtung 1 hier einen Befüllungsbereich 16 mit einem vorgelagerten Versorgungsportal 17 auf. Der Befüllungsbereich ist dabei durch das Fördersystem 18 mit dem Anlieferungsbereich 20 und den an dem Anlieferungsbereich 20 angrenzenden Futterrohstoffsilos 22, in denen angelieferter Futterrohstoff zur weiteren Verarbeitung oder Verwendung vorgehalten werden, sowie dem Lagerbereich 2 und dem Separationsbereich 14 verbunden. Zur Anlieferung etwaiger Materialien, insbesondere von Futterrohstoffen, verfügt der Anlieferungsbereich 20 hier über eine LKW-Rampe 21, womit ein einfaches Entladen eines mit entsprechenden Materialien gefüllten LKW's gewährleistet wird. Weiterhin können sich im Anlieferungsbereich etwaige Gerätschaften befinden, mit denen die angelieferten Futterrohstoffe aufbereitet bzw. verarbeitet werden können, bevor diese dann in die Futterrohstoffsilos 22 für die weitere Verarbeitung bzw. Verwendung vorgehalten werden.

Sind Kisten im Versorgungsportal 5 als erntereif identifiziert worden, so kann das Versorgungsportal 5 diese Kisten ausschleusen oder die Kisten werden aufgrund der empirischen Annahme, dass diese nunmehr erntereif sind, vom Regalbediengerät 4 direkt aus dem Lagerbereich 2 ausgeschleust. Die Versorgungsportale 5 als auch die Regalbediengeräte 4 können demnach beide jeweils erntereife Kisten aus dem Lagerbereich 2 ausschleusen und mittels des Fördersystems 18, welches ebenfalls mit dem Entleerungsbereich 11 verbunden ist, zum Entleerungsbereich 11 zur weiteren Behandlung transportieren. Im Entleerungsbereich 11 angekommen, können dort die als erntereif identifizierten Kisten mittels der Entleerungseinrichtung 12 entstapelt und entleert und die geleerten Kisten mittels der an die Entleerungseinrichtung 12 angeordneten Reinigungseinrichtung 13 gereinigt werden. Eine Reinigung muss allerdings nicht stattfinden. Denkbar ist auch die direkte Wiederverwendung der entleerten Kisten ohne vorherige Reinigung. Die durch die Reinigungseinrichtung 13 gereinigten Kisten können anschließend in einem Pufferbereich 15 für die weitere Verwendung, insbesondere der Neubefüllung oder zur Befüllung von Insekteneiern bzw. Insektenpuppen, vorgehalten werden.

Der mittels der Entleerungseinrichtung 12 entleerte Kisteninhalt kann anschließend durch das Fördersystem 18 vom Entleerungsbereich 11 in den Separationsbereich 14 befördert werden. Dort angelangt kann der entleerte Kisteninhalt in Produkte, Zwischenprodukte und sonstigem Kisteninhalt separiert werden, wobei Zwischenprodukte, also beispielsweise nicht vollständig ausgereifte Insekten, für die Wiederbestückung in den Befüllungsbereich 16 mittels des Fördersystems 18 zurück transportiert werden können. Die als Produkt abgetrennten Insekten, also beispielsweise vollständig reife Insekten oder Insekten mit einem bestimmten Reifegrad, können anschließend entweder als Lebendprodukt in Produktsilos 23 gelagert oder für die Weiterverarbeitung vorgehalten werden. Auch können die Lebendprodukte weiterverarbeitet werden, insbesondere zur Gewinnung von Öl und Mehl. Solche aus den Produkten gewonnen Endprodukte können ebenfalls in Produktsilos 23 gelagert werden, bevor diese beispielsweise durch einen LKW oder Güterzug zur weiteren Verarbeitung oder Verwendung an einen weiteren Ort transportiert werden. Der von den Zwischenprodukten und Produkten abgesonderte sonstige Kisteninhalt, insbesondere Insektenhäute und Insektenkot, kann beispielsweise zur Verwendung als oder zur Herstellung von Dünger dienen.

Darüber hinaus weist die in Figur 1 dargestellte Vorrichtung 1 einen Schlüpfbereich 7 und Puppenbearbeitungsbereich 9 mit einer Versorgungseinrichtung 8 und Separationseinrichtung 10 auf, in welcher Bearbeitungsschritte zur Optimierung der zur Aufzucht vorgesehenen Insekten selbsttätig und automatisiert durchgeführt werden können. Im Schlüpfbereich 7 können Behältnisse, insbesondere Kisten, welche gefüllt mit Insekteneiern und/oder Insektenpuppen sind, aufbewahrt und die Insekteneiern und/oder Insektenpuppen unter den optimalen Bedingungen innerhalb des Schlüpfbereichs 7 oder Puppenbearbeitungsbereichs 8 gebrütet werden. Bevorzugt weist der Schlüpfbereich 7 dafür einen Inkubator auf. Denkbar ist dabei auch, dass die mit Insekteneiern und/oder Insektenpuppen gefüllten Behältnisse im Hochregal 3 gelagert werden. Damit die aus den Eiern und/oder Puppen im Schlüpfbereich 7 und/oder Puppenbearbeitungsberiech oder im Hochregal 3 geschlüpften Larven und/oder Insekten versorgbar sind, weißt die Vorrichtung 1 eine Versorgungseinrichtung 8 auf, in welcher die geschlüpften Eier und/oder Puppen insbesondere mit Futter und Substrat für das optimale Wachstum versorgt werden können.

Damit die aus den Eiern im Schlüpfbereich 7 geschlüpften Larven transportiert und versorgt werden können, kann die Vorrichtung 1 darüber hinaus Transport-, Versorgungs-, Dosier- und Handhabungseinrichtung aufweisen, mit denen die frisch geschlüpften Larven und/oder Insekten für das optimale Wachstum mit dem notwendigen Materialien, insbesondere dem richtigen Futter, versorgt werden können. Die Bestückung mit Futter-, Substrat- oder Larvenportionen zur weiteren Aufzucht im Lagerbereich 2 kann entweder direkt durch die Versorgungseinrichtung 8 oder im Befüllungsbereich 16 stattfinden.

Haben die geschlüpften Larven und/oder Insekten ein entsprechendes Stadium für die weitere Aufzucht erreicht, können diese von gegebenenfalls ungeschlüpften Eiern bzw. Puppen, toten Insekten und etwaigen anderem Kisteninhalt durch die Separationseinrichtung 10 getrennt werden. Die so vom etwaigen sonstigem Kisteninhalt getrennten Junglarven bzw. jung Insekten können anschließend im Puppenbearbeitungsbereich 9 zunächst für die weitere Aufzucht behandelt oder durch die Beförderung mit dem Fördersystem 18 innerhalb des Befüllungsbereich 16 durch das vorgelagert Versorgungsportal 17 zusammen mit Materialien für die weitere Aufzucht in Kisten gefüllt und in den Lagerbereich 2 für die weitere Aufzucht transportiert werden.

Somit ist vorstehend eine Vorrichtung zur Aufzucht von Insekten in einem Hochregallager offenbart, dass eine automatisierte, ressourcenschonende und effiziente Aufzucht von Insekten ermöglicht.

### BEZUGSZEICHENLISTE

- 1: Vorrichtung
- 2: Lagerbereich
- 3: Hochregal
- 4: Regalbediengerät
- 5: Versorgungsportal

- 7: Schlüpfbereich
- 8: Versorgungseinrichtung
- 9: Puppenbearbeitungsbereich
- 10: Separationseinrichtung
- 11: Entleerungsbereich
- 12: Entleerungseinrichtung
- 13: Reinigungseinrichtung
- 14: Separationsbereich
- 15: Pufferbereich
- 16: Befüllungsbereich
- 17: vorgelagertes Versorgungsportal
- 18: Fördersystem
- 19: Plattform
- 20: Anlieferungsbereich
- 21: LKW-Rampe
- 22: Futterrohstoffsilo
- 23: Produktsilo

## Patentansprüche

1. Vorrichtung (1) zur Aufzucht von Insekten in einem Hochregallager, umfassend einen Lagerbereich (2) mit wenigstens einem Hochregal (3) und einer lagerlogistischen Einrichtung, wobei in dem wenigstens einen Hochregal (3) Kisten stapelbar lagerbar sind und die Kisten mittels der lagerlogistischen Einrichtung innerhalb des Lagerbereichs (2) transportierbar und/oder ausschleusbar sind, wobei sich in den Kisten Insekten befinden,
wobei die Vorrichtung (1) (3) ein Versorgungsportal (5) aufweist, wobei mittels der lagerlogistischen Einrichtung die im Hochregal (3) stapelbar lagerbaren Kisten aus dem wenigstens einen Hochregal (3) zu dem Versorgungsportal (5) transportierbar und der Zustand des Kisteninhalts innerhalb der Kisten mittels des Versorgungsportals (5) bestimmbar und die Insekten in den Kisten mit dem Versorgungsportal (5) versorgbar und/oder die Kisten ausschleusbar sind,
**dadurch gekennzeichnet, dass** das Versorgungsportal (5) an dem wenigstens einen Hochregal (3) angeordnet ist.

2. Vorrichtung nach Anspruch 1, **dadurch gekennzeichnet, dass** das Versorgungsportal (5) wenigstens einen Pufferbereich aufweist, in welchem die von der lagerlogistischen Einrichtung zum Versorgungsportal (5) transportierten Kisten zwischenlagerbar sind.

3. Vorrichtung nach Anspruch 1 oder 2, **dadurch gekennzeichnet, dass** das Versorgungsportal (5) wenigstens eine Detektionseinrichtung aufweist, mit welcher die für die Insektenzucht benötigten Parameter, insbesondere Feuchtigkeit und Temperatur, innerhalb einer Kiste feststellbar sind, wobei bevorzugt die Detektionseinrichtung wenigstens einen Sensor und/oder eine Vorrichtung zur Aufzeichnung und/oder Datenübermittlung, insbesondere eine Kamera, aufweist.

4. Vorrichtung nach Anspruch 3, **dadurch gekennzeichnet, dass** die durch die Detektionseinrichtung ermittelten Parameter, die den Zustand des Inhalts in einer der Kisten wiederspiegeln, mit einer Referenzdatenbank vergleichbar sind, sodass insbesondere der jeweilige Zustand des Kisteninhalts und/oder Reifegrad der Insekten innerhalb einer Kiste feststellbar ist, wobei bevorzugt die durch den Vergleich der ermittelten Parameter mit den Referenzdatenbank erhaltenen Daten zum maschinellen Lernen der Detektionseinrichtung verwendbar sind und besonders bevorzugt die erhaltenen Daten zur automatisierten Optimierung der Produktionsparameter, insbesondere der Rezeptur und Dosierung des Futtermittels, nutzbar sind.

5. Vorrichtung nach wenigstens einem der vorhergehenden Ansprüche, **dadurch gekennzeichnet, dass** das Versorgungsportal (5) wenigstens ein Versorgungsmodul aufweist, mit welchem die Insekten in einer Kiste mit Versorgungsmaterialien, insbesondere Futtermittel und Substrat, versorgbar sind.

6. Vorrichtung nach wenigstens einem der vorhergehenden Ansprüche, **dadurch gekennzeichnet, dass** das Versorgungsportal (5) wenigstens ein Reinigungsmodul aufweist, mit welchem Teile des Kisteninhalts, insbesondere Häute der Insekten, Kot und Futtermittelreste, aus einer Kiste entfernbar sind, wobei das Reinigungsmodul bevorzugt wenigstens eine Saugvorrichtung aufweist, mit welcher durch Absaugen oder Windsichten die zu entfernenden Teile des Kisteninhalts aus einer Kiste entfernbar sind und/oder wenigstens eine elektrische Einrichtung aufweist, mit welcher der zu entfernende Teil des Kisteninhalts durch Elektrostatik aus einer Kiste entfernbar ist.

7. Vorrichtung nach wenigstens einem der vorhergehenden Ansprüche, **dadurch gekennzeichnet, dass** diese wenigstens einen Schlüpfbereich (7) aufweist, wobei bevorzugt der Schlüpfbereich (7) einen Inkubator zum Brüten von Insekteneier und/oder Insektenpuppen aufweist.

8. Vorrichtung nach wenigstens einem der vorhergehenden Ansprüche, **dadurch gekennzeichnet, dass** diese wenigstens einen Puppenbearbeitungsbereich (9) aufweist.

9. Vorrichtung nach wenigstens einem der Anspruch 7 oder 8, dass diese wenigstens eine Separationseinrichtung (10) aufweist, mit welchem die Insekteneier und/oder Insektenpuppen oder die im Schlüpfbereich (7) und/oder Puppenbearbeitungsbereich (9) und/oder im Hochregallager geschlüpften Larven und/oder Insekten vom sonstigen Kisteninhalt abtrennbar sind und/oder diese eine Versorgungseinrichtung (10) aufweist, mit dem die Insekteneier, Insektenpuppen, geschlüpften Larven und/oder Insekten mit Versorgungsmaterial versorgbar sind.

10. Vorrichtung nach wenigstens einem der vorhergehenden Ansprüche, **dadurch gekennzeichnet, dass** wenigstens ein Entleerungsbereich (11) vorhanden ist, in welchem die Kisten entstapelbar und der Kisteninhalt aus den Kisten entfernbar ist, wobei bevorzugt der Entleerungsbereich (11) eine Entleerungseinrichtung (12) aufweist, mit welchem die Entstapelung der Kisten und die Entfernung des Kisteninhalts aus den Kisten durchführbar ist, wobei besonders bevorzugt der Entleerungsbereich (11) wenigstens eine Reinigungseinrichtung (13) aufweist, mit welcher die entstapelten und entleerten Kisten reinigbar sind.

11. Vorrichtung nach Anspruch 10, **dadurch gekennzeichnet, dass** wenigstens ein Separationsbereich (14) vorhanden ist, in welchem der aus den Kisten entleerte Kisteninhalt in Produkte, Zwischenprodukte und sonstigem Kisteninhalt trennbar ist, wobei bevorzugt der aus den Kisten entleerte Kisteninhalt in Form eines Schüttguts zu dem Separationsbereich (14) transportierbar ist.

12. Vorrichtung nach wenigstens einem der Ansprüche 10 oder 11, **dadurch gekennzeichnet, dass** wenigstens ein Pufferbereich (15) vorhanden ist, in welchem leere und/oder gereinigte Kisten vorhaltbar sind.

13. Vorrichtung nach wenigstens einem der Ansprüche, **dadurch gekennzeichnet, dass** diese wenigstens einen Befüllungsbereich (16) aufweist, in welchem die Kisten mit Futtermittel, Insekten, Insekteneiern oder einer Kombination hiervon befüllbar sind, wobei der Befüllungsbereich (16) bevorzugt wenigstens ein vorgelagertes Versorgungsportal (17) zur Befüllung der Kisten aufweist und besonders bevorzugt für wenigstens ein Stadium der Insekten jeweils ein vorgelagertes Versorgungsportal (17) vorhanden ist sowie bevorzugt der Befüllungsbereich wenigstens eine Befüllungsstraße aufweist, in welcher die Befüllung der Kisten stattfindet.

14. Vorrichtung nach wenigstens einem der vorhergehenden Ansprüche, **dadurch gekennzeichnet, dass** diese wenigstens ein Fördersystem (18) aufweist, mit welchem insbesondere die Kisten oder gestapelte oder palettierte Kistenstapel aus und in den Lagerbereich (2) beförderbar sind, wobei bevorzugt das Fördersystem (18) ganz oder teilweise ein fahrerloses Transportsystem ist.

15. Vorrichtung nach wenigstens einem der vorhergehenden Ansprüche, **dadurch gekennzeichnet, dass** diese wenigstens einen Anlieferungsbereich (20) aufweist, an welche Materialien, insbesondere Futtermittel für die Aufzucht der Insekten, anlieferbar sind, wobei bevorzugt an den Anlieferungsbereich (20) wenigstens ein Futterrohstoffsilo (22) angeordnet ist.

16. Vorrichtung nach wenigstens einem der vorhergehenden Ansprüche, **dadurch gekennzeichnet, dass** diese wenigstens ein Produktsilo (23) aufweist, in welchem ein aus der Aufzucht der Insekten gewonnenes Produkt lagerbar ist und/oder der Lagerbereich (2) wenigstens eine Klimatisierungseinrichtung aufweist, mit welcher die Lufteigenschaften innerhalb des Lagerbereichs (2) regulierbar sind und/oder der Lagerbereich (2) an dem wenigstens einem, an dem Hochregal (3) angeordneten Versorgungsportals (5) wenigstens eine Plattform (19) zur Wartung, zum manuellen Eingreifen und zum manuellen Bestücken des Versorgungsportals (5) aufweist und/oder der Lagerbereich (2) in Silobauweise ausgeführt ist, sodass das wenigstens eine Hochregal (3) als Tragelement für wenigstens eine Außenfassade dient, wobei bevorzugt das wenigstens eine als Tragelement für wenigstens eine Außenfassade dienende Hochregal (3) eine Isolierung mit einer Dampfsperre aufweist, sodass der Lagerbereich (2) nach außen hin abgeschirmt ist.

17. Verfahren zur Aufzucht von Insekten in einem Hochregallager mit einer Vorrichtung gemäß wenigstens einem der Ansprüche 1 bis 16, **dadurch gekennzeichnet, dass** ein Stapel von Kisten aus wenigstens einem Hochregal (3) entnommen und zu einem Versorgungsportal (5) transportiert wird, wobei für jede der in das Versorgungsportal (5) transportierten Kiste der Zustand des Kisteninhalts der jeweiligen Kiste mittels des Versorgungsportals (5) bestimmt und abhängig von dem bestimmten Zustand die Kisten ausgeschleust oder versorgt werden, wobei nach Versorgung die jeweiligen Kisten in das wenigstens eine Hochregal (3) zurück transportiert werden.

18. Verfahren nach Anspruch 17, **dadurch gekennzeichnet, dass** die in und aus dem wenigstens einen Hochregal (3) transportierten Kisten nach einem Chaosprinzip oder einer anderen Form der Lageroptimierung in dem wenigstens einen Hochregal (3) gelagert werden.

19. Verfahren nach Anspruch 17 oder 18, **dadurch gekennzeichnet, dass** die zu dem Versorgungsportal (5) transportierten Kisten von diesem Versorgungsportal (5) zu einem anderem Versorgungsportal transportiert werden.

20. Verfahren nach wenigstens einem der Ansprüche 17 bis 19, **dadurch gekennzeichnet, dass** bei erntereifen Kisten das Substrat und/oder Futtermittel entfernt wird, sodass die Insekten ihren Darm entleeren, wobei bevorzugt die Entfernung des Substrats und/oder Futtermittel 1 bis 5 Tage vor dem Ausschleusen der erntereifen Kiste durchgeführt wird.

21. Verfahren nach wenigstens einem der vorhergehenden Ansprüche 17 bis 20, **dadurch gekennzeichnet, dass** ausgeschleuste, erntereife Kisten entstapelt und entleert werden, wobei bevorzugt die entstapelten und entleerten Kisten gereinigt werden, wobei besonders bevorzugt die Kisten gelagert und für die weitere Verwendung vorgehalten werden.

22. Verfahren nach Anspruch 21, **dadurch gekennzeichnet, dass** der entleerte Kisteninhalt in Produkte, Zwischenprodukte und sonstigem Kisteninhalt separiert wird, wobei bevorzugt die Zwischenprodukte in Kisten zur weiteren Aufzucht eingefüllt werden, wobei besonders bevorzugt das Produkt als Lebendprodukt erhalten oder inaktiviert und geschreddert wird, wobei bevorzugt das geschredderte Produkt entfettet und ein Öl gebildet wird, wobei besonders bevorzugt das entfette Produkt getrocknet und gemahlen wird, sodass ein Mehl gebildet wird.

## Claims

1. Apparatus (1) for rearing insects in a high-bay warehouse, comprising a storage region (2) having at least one high-bay rack (3) and a storage logistics device, it being possible for the boxes to be stored in a stackable manner in the at least one high-bay rack (3) and for the boxes to be transported and/or discharged within the storage region (2) by means of the storage logistics device, insects being located in the boxes, the apparatus (1) (3) having a supply portal (5), it being possible for the boxes, which can be stored in a stackable manner in the high-bay rack (3), to be transported from the at least one high-bay rack (3) to the supply portal (5) by means of the storage logistics device and the state of the box contents within the boxes to be determined by means of the supply portal (5) and the insects in the boxes to be supplied using the supply portal (5) and/or the boxes to be discharged, **characterized in that** the supply portal (5) is arranged on the at least one high-bay rack (3).

2. Apparatus according to claim 1, **characterized in that** the supply portal (5) has at least one buffer region in which the boxes transported from the storage logistics device to the supply portal (5) can be temporarily stored.

3. Apparatus according to claim 1 or 2, **characterized in that** the supply portal (5) has at least one detection device by means of which the parameters required for the insect breeding, in particular humidity and temperature, can be determined within a box, the detection device preferably having at least one sensor and/or an apparatus for recording and/or data transmission, in particular a camera.

4. Apparatus according to claim 3, **characterized in that** the parameters determined by the detection device, which reflect the state of the contents in one of the boxes, can be compared with a reference database, so that in particular the particular state of the box contents and/or degree of maturity of the insects within a box can be determined, it preferably being possible for the data obtained by comparing the determined parameters with the reference database to be used for the machine learning of the detection device and particularly preferably for the data obtained to be used for the automated optimization of the production parameters, in particular the recipe and dosage of the feed.

5. Apparatus according to at least one of the preceding claims, **characterized in that** the supply portal (5) has at least one supply module by means of which the insects in a box can be supplied with supply materials, in particular feed and substrate.

6. Apparatus according to at least one of the preceding claims, **characterized in that** the supply portal (5) has at least one cleaning module by means of which parts of the box contents, in particular insect skins, excrement and feed residues, can be removed from a box, the cleaning module preferably having at least one suction apparatus by means of which the parts of the box contents to be removed can be removed from a box by suction or air classification and/or having at least one electrical device by means of which the part of the box contents to be removed can be removed from a box by electrostatics.

7. Apparatus according to at least one of the preceding claims, **characterized in that** the apparatus has at least one hatching region (7), the hatching region (7) preferably having an incubator for breeding insect eggs and/or insect pupae.

8. Apparatus according to at least one of the preceding claims, **characterized in that** the apparatus has at least one pupae processing region (9).

9. Apparatus according to at least one of claims 7 or 8, that the apparatus has at least one separation device (10) by means of which the insect eggs and/or insect pupae or the larvae and/or insects hatched in the hatching region (7) and/or pupae processing region (9) and/or in the high-bay warehouse can be separated from the other box contents and/or the apparatus has a supply device (10) by means of which the insect eggs, insect pupae, hatched larvae and/or insects can be supplied with supply material.

10. Apparatus according to at least one of the preceding claims, **characterized in that** there is at least one emptying region (11) in which the boxes can be unstacked and the box contents can be removed from the boxes, the emptying region (11) preferably having an emptying device (12) by means of which the unstacking of the boxes and the removal of the box contents from the boxes can be carried out, the emptying region (11) particularly preferably having at least one cleaning device (13) by means of which the unstacked and emptied boxes can be cleaned.

11. Apparatus according to claim 10, **characterized in that** there is at least one separation region (14) in which the box contents emptied from the boxes can be separated into products, intermediate products and other box contents, it preferably being possible for the box contents emptied from the boxes to be transported to the separation region (14) in the form of bulk material.

12. Apparatus according to at least one of claims 10 or 11,
**characterized in that** there is at least one buffer region (15) in which empty and/or cleaned boxes can be kept available.

13. Apparatus according to at least one of the claims, **characterized in that** the apparatus has at least one filling region (16) in which the boxes can be filled with feed, insects, insect eggs or a combination thereof, the filling region (16) preferably having at least one upstream supply portal (17) for filling the boxes and there particularly preferably being an upstream supply portal (17) for at least one stage of the insects and the filling region preferably having at least one filling line in which the filling of the boxes takes place.

14. Apparatus according to at least one of the preceding claims, **characterized in that** the apparatus has at least one conveyor system (18) by means of which in particular the boxes or stacked or palletized stacks of boxes can be conveyed out of and into the storage region (2), the conveyor system (18) preferably being a completely or partially driverless transport system.

15. Apparatus according to at least one of the preceding claims, **characterized in that** the apparatus has at least one delivery region (20) to which materials, in particular feed for rearing the insects, can be delivered, at least one feed raw material silo (22) preferably being arranged at the delivery region (20).

16. Apparatus according to at least one of the preceding claims, **characterized in that** the apparatus has at least one product silo (23) in which a product obtained from the rearing of the insects can be stored and/or the storage region (2) has at least one air conditioning device by means of which the air properties within the storage region (2) can be regulated and/or the storage region (2), on the at least one supply portal (5) arranged on the high-bay rack (3), has at least one platform (19) for maintenance, for manual intervention and for manually loading the supply portal (5) and/or the storage region (2) is designed in silo construction, so that the at least one high-bay rack (3) is used as a support element for at least one external facade, the at least one high-bay rack (3) being used as a support element for at least one external facade preferably having insulation with a vapor barrier, so that the storage region (2) is shielded from the outside.

17. Method for rearing insects in a high-bay warehouse comprising an apparatus according to at least one of claims 1 to 16, **characterized in that** a stack of boxes is taken from at least one high-bay rack (3) and transported to a supply portal (5), the state of the box contents of the particular box being determined for each of the boxes transported into the supply portal (5) by means of the supply portal (5) and the boxes being discharged or supplied depending on the determined state thereof, the respective boxes being transported back into the at least one high-bay rack (3) after the supply.

18. Method according to claim 17, **characterized in that** the boxes transported into and out of the at least one high-bay rack (3) are stored in the at least one high-bay rack (3) according to a chaos principle or another form of storage optimization.

19. Method according to claim 17 or 18, **characterized in that** the boxes transported to the supply portal (5) are transported from this supply portal (5) to another supply portal.

20. Method according to at least one of claims 17 to 19, **characterized in that** in the case of harvest-ready boxes, the substrate and/or feed is removed so that the insects empty their intestines, the removal of the substrate and/or feed preferably being carried out 1 to 5 days before the harvest-ready box is discharged.

21. Method according to at least one of the preceding claims 17 to 20, **characterized in that** discharged, harvest-ready boxes are unstacked and emptied, the unstacked and emptied boxes preferably being cleaned, the boxes particularly preferably being stored and kept available for further use.

22. Method according to claim 21, **characterized in that** the emptied box contents are separated into products, intermediate products and other box contents, the intermediate products preferably being filled into boxes for further breeding, the product particularly preferably being maintained as a live product or inactivated and shredded, the shredded product preferably being defatted and an oil being formed, the defatted product particularly preferably being dried and ground so that a flour is formed.

## Revendications

1. Dispositif (1) permettant l'élevage d'insectes dans un entrepôt à chambres très hautes, comprenant une zone d'entreposage (2) comportant au moins une chambre très haute (3) et un appareil logistique d'entreposage, dans lequel des caisses peuvent être entreposées de manière empilable dans l'au moins une chambre très haute (3) et les caisses peuvent être transportées et/ou évacuées au moyen de l'appareil logistique d'entreposage à l'intérieur de la zone d'entreposage (2), dans lequel des insectes se trouvent dans les caisses, dans lequel le dispositif (1) (3) présente un portail d'alimentation (5), dans lequel, au moyen de l'appareil logistique d'entreposage, les caisses pouvant être entreposées de manière empilable dans la chambre très haute (3) peuvent être transportées hors de l'au moins une chambre très haute (3) vers le portail d'alimentation (5) et l'état du contenu de caisse à l'intérieur des caisses peut être déterminé au moyen du portail d'alimentation (5) et les insectes dans les caisses peuvent être alimentés par le portail d'alimentation (5) et/ou les caisses peuvent être évacuées, **caractérisé en ce que** le portail d'alimentation (5) est disposé sur l'au moins une chambre très haute (3).

2. Dispositif selon la revendication 1, **caractérisé en ce que** le portail d'alimentation (5) présente au moins une zone tampon dans laquelle les caisses transportées par l'appareil logistique d'entreposage vers le portail d'alimentation (5) peuvent être entreposées temporairement de manière provisoire.

3. Dispositif selon la revendication 1 ou 2, **caractérisé en ce que** le portail d'alimentation (5) présente au moins un appareil de détection avec lequel les paramètres nécessaires à l'élevage d'insectes, en particulier l'humidité et la température, peuvent être constatés à l'intérieur d'une caisse, dans lequel, de préférence, l'appareil de détection présente au moins un capteur et/ou un dispositif permettant l'enregistrement et/ou la transmission de données, en particulier une caméra.

4. Dispositif selon la revendication 3, **caractérisé en ce que** les paramètres établis par l'appareil de détection reflétant l'état du contenu dans l'une des caisses peuvent être comparés à une base de données de référence, de sorte qu'en particulier l'état respectif du contenu de caisse et/ou le degré de maturité des insectes à l'intérieur d'une caisse peuvent être constatés, dans lequel, de préférence, les données obtenues par la comparaison des paramètres établis avec la base de données de référence peuvent être utilisées pour l'apprentissage automatique de l'appareil de détection et, de manière particulièrement préférée, les données obtenues peuvent être utilisées pour l'optimisation automatisée des paramètres de production, en particulier la recette et le dosage des aliments.

5. Dispositif selon au moins l'une des revendications précédentes, **caractérisé en ce que** le portail d'alimentation (5) présente au moins un module d'alimentation avec lequel les insectes dans une caisse peuvent être alimentés en matériaux d'alimentation, en particulier en aliments et en substrat.

6. Dispositif selon au moins l'une des revendications précédentes, **caractérisé en ce que** le portail d'alimentation (5) présente au moins un module de nettoyage avec lequel des parties du contenu de caisse, en particulier des peaux d'insectes, des excréments et des restes d'aliments, peuvent être éliminées d'une caisse, dans lequel le module de nettoyage présente de préférence au moins un dispositif d'aspiration avec lequel les parties à éliminer du contenu de caisse peuvent être éliminées d'une caisse par aspiration ou par séparation par soufflage et/ou présente au moins un appareil électrique avec lequel la partie à éliminer du contenu de caisse peut être éliminée d'une caisse par électrostatique.

7. Dispositif selon au moins l'une des revendications précédentes, **caractérisé en ce que** ledit dispositif présente au moins une zone d'éclosion (7), dans lequel, de préférence, la zone d'éclosion (7) présente un incubateur pour l'incubation d'œufs d'insectes et/ou de pupes d'insectes.

8. Dispositif selon au moins l'une des revendications précédentes, **caractérisé en ce que** ledit dispositif présente au moins une zone de traitement des pupes (9).

9. Dispositif selon au moins l'une des revendications 7 ou 8, **en ce que** ledit dispositif présente au moins un appareil de séparation (10) avec lequel les œufs d'insectes et/ou les pupes d'insectes ou les larves et/ou les insectes éclos dans la zone d'éclosion (7) et/ou dans la zone de traitement des pupes (9) et/ou dans l'entrepôt à chambres très hautes peuvent être séparés d'autres contenus de caisse et/ou ledit dispositif présente un appareil d'alimentation (10) avec lequel les œufs d'insectes, pupes d'insectes, larves et/ou insectes éclos peuvent être alimentés en matériau d'alimentation.

10. Dispositif selon au moins l'une des revendications précédentes, **caractérisé en ce qu'**au moins une zone de vidage (11) est prévue, dans laquelle les caisses peuvent être dépilées et le contenu de caisse peut être éliminé des caisses, dans lequel la zone de vidage (11) présente de préférence un appareil de vidage (12) avec lequel le dépilage des caisses et l'élimination du contenu de caisse peuvent être mis en œuvre, dans lequel la zone de vidage (11) présente de manière particulièrement préférée au moins un appareil de nettoyage (13) avec lequel les caisses dépilées et vidées peuvent être nettoyées.

11. Dispositif selon la revendication 10, **caractérisé en ce qu'**au moins une zone de séparation (14) est prévue, dans laquelle le contenu de caisse vidé des caisses peut être séparé en produits, produits intermédiaires et autre contenu de caisse, dans lequel le contenu de caisse vidé des caisses peut de préférence être transporté vers la zone de séparation (14) sous forme de produit en vrac.

12. Dispositif selon au moins l'une des revendications 10 ou 11, **caractérisé en ce qu'**au moins une zone tampon (15) est prévue, dans laquelle des caisses vides et/ou nettoyées peuvent être conservées.

13. Dispositif selon au moins l'une des revendications, **caractérisé en ce que** ledit dispositif présente au moins une zone de remplissage (16) dans laquelle les caisses peuvent être remplies d'aliments, insectes, œufs d'insectes ou d'une combinaison de ceux-ci, dans lequel la zone de remplissage (16) présente, de préférence, au moins un portail d'alimentation (17) entreposé en amont pour le remplissage des caisses et, de manière particulièrement préférée, respectivement un portail d'alimentation (17) entreposé en amont est prévu pour au moins une phase des insectes, et la zone de remplissage présente de préférence au moins une ligne de remplissage dans laquelle a lieu le remplissage des caisses.

14. Dispositif selon au moins l'une des revendications précédentes,
**caractérisé en ce que** ledit dispositif présente au moins un système de transport (18) avec lequel les caisses ou les piles de caisses empilées ou palettisées peuvent en particulier être transportées hors de la zone d'entreposage (2) et dans celle-ci, dans lequel le système de transport (18) est de préférence entièrement ou partiellement un système de transport sans conducteur.

15. Dispositif selon au moins l'une des revendications précédentes, **caractérisé en ce que** ledit dispositif présente au moins une zone de livraison (20) au niveau de laquelle des matériaux, en particulier des aliments pour l'élevage des insectes, peuvent être livrés, dans lequel au moins un silo de matières premières alimentaires (22) est de préférence disposé sur la zone de livraison (20).

16. Dispositif selon au moins l'une des revendications précédentes, **caractérisé en ce que** ledit dispositif présente au moins un silo à produits (23) dans lequel un produit obtenu à partir de l'élevage des insectes peut être entreposé et/ou la zone d'entreposage (2) présente au moins un appareil de climatisation avec lequel les propriétés de l'air peuvent être régulées à l'intérieur de la zone d'entreposage (2) et/ou la zone d'entreposage (2) présente, sur l'au moins un portail d'alimentation (5) disposé sur la chambre très haute (3), au moins une plate-forme (19) pour l'entretien, pour l'intervention manuelle et pour l'équipement manuel du portail d'alimentation (5) et/ou la zone d'entreposage (2) est réalisée sous forme de construction en silo, de sorte que l'au moins une chambre très haute (3) sert d'élément porteur pour au moins une façade extérieure, dans lequel, de préférence, l'au moins une chambre très haute (3) servant d'élément porteur pour au moins une façade extérieure présente une isolation comportant un pare-vapeur, de sorte que la zone d'entreposage (2) est protégée de l'extérieur.

17. Procédé permettant l'élevage d'insectes dans un entrepôt à chambres très hautes comportant un dispositif selon au moins l'une des revendications 1 à 16, **caractérisé en ce qu'**une pile de caisses est prélevée d'au moins une chambre très haute (3) et est transportée vers un portail d'alimentation (5), dans lequel pour chaque caisse transportée dans le portail d'alimentation (5), l'état du contenu de caisse de la caisse respective est déterminé au moyen du portail d'alimentation (5) et, en fonction de l'état déterminé, les caisses sont évacuées ou alimentées, dans lequel, après alimentation, les caisses respectives sont transportées de nouveau dans l'au moins une chambre très haute (3).

18. Procédé selon la revendication 17, **caractérisé en ce que** les caisses transportées dans l'au moins une chambre très haute (3) et hors de celle-ci sont entreposées dans l'au moins une chambre très haute (3) selon un principe chaotique ou une autre forme d'optimisation d'entreposage.

19. Procédé selon la revendication 17 ou 18, **caractérisé en ce que** les caisses transportées vers le portail d'alimentation (5) sont transportées depuis ledit portail d'alimentation (5) vers un autre portail d'alimentation.

20. Procédé selon au moins l'une des revendications 17 à 19,
**caractérisé en ce que,** en présence de caisses prêtes à être récoltées, le substrat et/ou les aliments sont éliminés de sorte que les insectes vident leurs boyaux, dans lequel, de préférence, l'élimination du substrat et/ou des aliments est effectuée 1 à 5 jours avant l'évacuation de la caisse prête à être récoltée.

21. Procédé selon au moins l'une des revendications précédentes 17 à 20, **caractérisé en ce que** des caisses prêtes à être récoltées et évacuées sont dépilées et vidées, dans lequel, de préférence, les caisses dépilées et vidées sont nettoyées, dans lequel, de manière particulièrement préférée, les caisses sont entreposées et réservées pour une utilisation ultérieure.

22. Procédé selon la revendication 21, **caractérisé en ce que** le contenu de caisse vidé est séparé en produits, produits intermédiaires et autre contenu de caisse, dans lequel, de préférence, les produits intermédiaires sont introduits dans des caisses pour un élevage ultérieur, dans lequel, de manière particulièrement préférée, le produit est obtenu sous forme de produit vivant ou est inactivé et déchiqueté, dans lequel, de préférence, le produit déchiqueté est dégraissé et une huile est formée, dans lequel, de manière particulièrement préférée, le produit dégraissé est séché et broyé de manière à former une farine.
